(19) Europäisches Patentamt
European Patent Office
Office européen des brevets

(11) Veröffentlichungsnummer: **0 384 193 A2**

(12) # EUROPÄISCHE PATENTANMELDUNG

(21) Anmeldenummer: **90102142.8**

(51) Int. Cl.[5]: **C07D 213/61, A61K 31/44**

(22) Anmeldetag: **03.02.90**

(30) Priorität: **18.02.89 DE 3905028**

(43) Veröffentlichungstag der Anmeldung:
**29.08.90 Patentblatt 90/35**

(84) Benannte Vertragsstaaten:
**AT BE CH DE DK ES FR GB IT LI NL SE**

(71) Anmelder: **BAYER AG**
**D-5090 Leverkusen 1 Bayerwerk(DE)**

(72) Erfinder: **Lindel, Hans, Dr.**
**Carl-Duisberg-Strasse 321**
**D-5090 Leverkusen 1(DE)**
Erfinder: **Hallenbach, Werner, Dr.**
**Lichtenberger Strasse 68**
**D-4019 Monheim(DE)**
Erfinder: **Berschauer, Friedrich, Prof. Dr.**
**Bauermannskulle 66**
**D-5650 Solingen 1(DE)**
Erfinder: **Greife, Heinrich, Prof. Dr.**
**Salmstrasse 23**
**D-4018 Langenfeld(DE)**
Erfinder: **Klotz, Gernot, Dr.**
**Förster-Sons-Strasse 32**
**D-5653 Leichlingen 1(DE)**

(54) **2,4-Dihalogen-6-pyridylethanolphenylisopropylamine, Verfahren zu ihrer Herstellung und ihre Verwendung zur Verschiebung des Protein-Fett-Verhältnisses zugunsten von Protein.**

(57) Gegenstand der vorliegenden Erfindung sind neue 2,4-Dihalogen-6-pyridyl-ethanolamine der Formel I

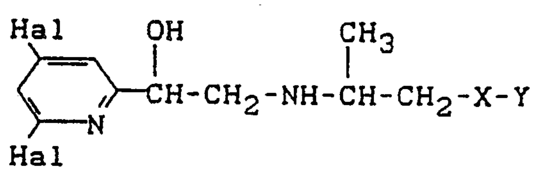

(I)

in welcher
Hal gleich oder verschieden für Fluor, Chlor, Brom stehen,
X für eine direkte Bindung oder $-CH_2-$ steht,
Y für Phenyl steht das durch OH, $C_{1-4}$-Alkoxy, $C_{1-4}$-Alkoxy-$C_{1-4}$-alkoxy, Carboxyl, Aminocarbonyl, $C_{1-4}$-Alkoxycarbonyl, Hydroxy-$C_{1-4}$-alkoxy, $C_{1-4}$-Alkoxycarbonyl-$C_{1-4}$-alkoxy, Phenyl-$C_{1-4}$-alkoxy substituiert ist sowie ihre physiologisch verträglichen Salze und N-Oxide, ihre Herstellung und Zwischenprodukte zu ihrer Herstellung sowie ihre Verwendung zur Verschiebung des Protein-Fett-Verhältnisses zugunsten von Protein sowie zur Behandlung der Adipositas bei Mensch und Tier.

EP 0 384 193 A2

**2,4-Dihalogen-6-pyridylethanolphenylisopropylamine, Verfahren zu ihrer Herstellung und ihre Verwendung zur Verschiebung des Protein-Fett-Verhältnisses zugunsten von Protein**

Die vorliegende Erfindung betrifft neue 2,4-Dihalogen-6-pyridylethanolphenylisopropylamine, Verfahren zu ihrer Herstellung und ihre Verwendung zur Verschiebung des Protein-Fett-Verhältnisses zugunsten von Protein sowie zur Behandlung der Adipositas bei Mensch und Tier.

2-Halogen-6-pyridylethanolphenylisopropylamine sind bereits bekannt. Sie eignen sich als Leistungsförderer bei Tieren (EP-OS 170 538, 254 856, 256 420).

So sind z. B. aus EP-OS 254 856 folgende Verbindungen bekannt:

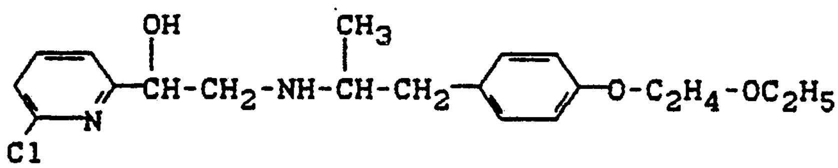

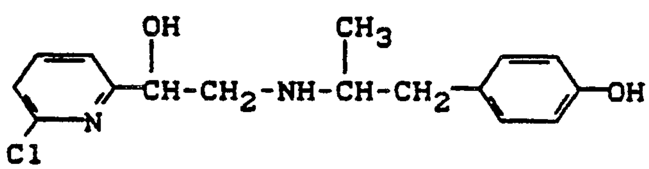

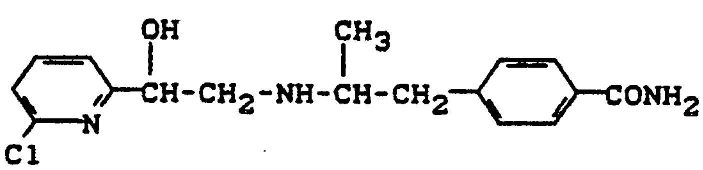

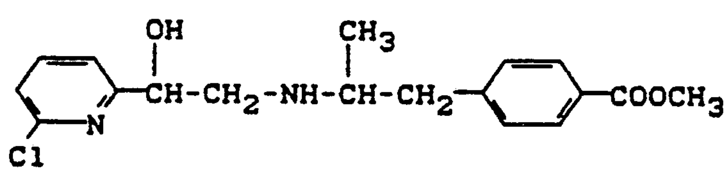

Diese Verbindungen wirken lipolytisch, zeigen aber starken steigernden Einfluß auf die Herzfrequenz beim Hund. Ihre Verwendung als Mittel zur Beeinflußung des Fettstoffwechsels ist durch diese Wirkung auf den Kreislauf behindert.

Gegenstand der vorliegenden Erfindung sind

1. 2,4-Dihalogen-6-pyridyl-ethanolamine der Formel I

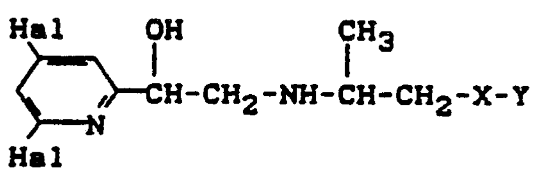 (I)

in welcher

Hal gleich oder verschieden für Fluor, Chlor, Brom stehen,

X für eine direkte Bindung oder $-CH_2-$ steht,
Y für Phenyl steht das durch OH, $C_{1-4}$-Alkoxy, $C_{1-4}$-Alkoxy-$C_{1-4}$-alkoxy, Carboxyl, Aminocarbonyl, $C_{1-4}$-Alkoxycarbonyl, Hydroxy-$C_{1-4}$-alkoxy, $C_{1-4}$-Alkoxycarbonyl-$C_{1-4}$-alkoxy, Phenyl-$C_1$-$C_4$-alkoxy substituiert ist
sowie ihre physiologisch verträglichen Salze und N-Oxide.

2. Verfahren zur Herstellung der Verbindungen der Formel I,

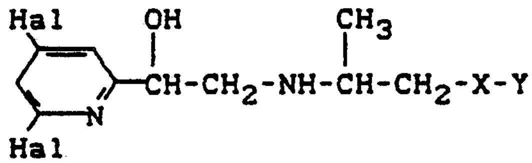

(I)

in welcher
Hal gleich oder verschieden für Fluor, Chlor, Brom stehen,
X für eine direkte Bindung oder $-CH_2-$ steht,
Y für Phenyl steht das durch OH, $C_{1-4}$-Alkoxy, $C_{1-4}$-Alkoxy-$C_{1-4}$-alkoxy, Carboxyl, Aminocarbonyl, $C_{1-4}$-Alkoxycarbonyl, Hydroxy-$C_{1-4}$-alkoxy, $C_{1-4}$-Alkoxycarbonyl-$C_{1-4}$-alkoxy, Phenyl-$C_1$-$C_4$-alkoxy substituiert ist,

a) indem man Halogenmethylketone der Formel II

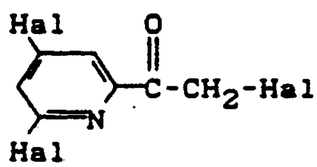

(II)

in welcher
Hal für Halogen steht,
mit Aminen der Formel III

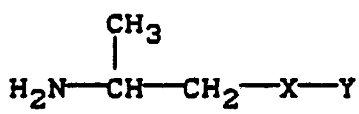

(III)

in welcher
X und Y die oben angegebene Bedeutung haben,
umsetzt und anschließend die Carbonylgruppe reduziert, oder

b) indem man Epoxide der Formel IV

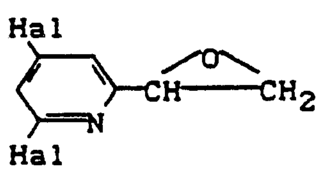

(IV)

in welcher
Hal für Halogen steht,
$R^1$, $R^2$, $R^3$ die oben angegebene Bedeutung haben,
mit Aminen der Formel III

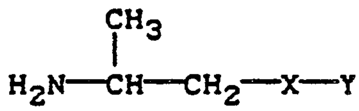

(III)

in welcher
X und Y die oben angegebene Bedeutung haben,
umsetzt, oder

c) indem man β-Halogenethylverbindungen der Formel V

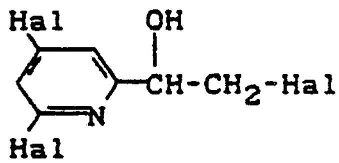

(V)

in welcher
Hal für Halogen steht,
mit Aminen der Formel III

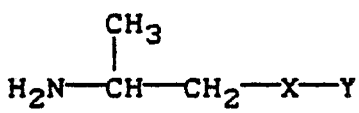

(III)

in welcher
X und Y die oben angegebene Bedeutung haben,
umsetzt, oder

d) Verbindungen der Formel VI

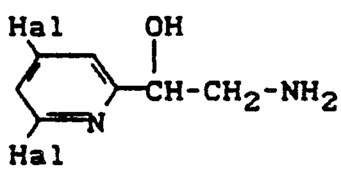

(VI)

in welcher
Hal für Halogen steht,
mit Ketonen der Formel VII

$CH_3-\overset{O}{\overset{\|}{C}}-CH_2-X-Y$ (VII)

in welcher
X und Y die oben angegebene Bedeutung haben,
unter reduzierenden Bedingungen umsetzt, oder

e) indem man Verbindungen der Formel VIII

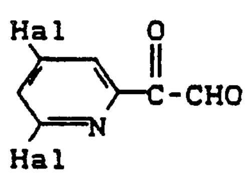

(VIII)

in welcher
Hal für Halogen steht,

mit Aminen der Formel III

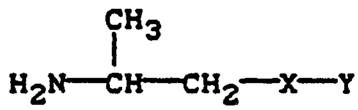

(III)

in welcher X und Y die oben angegebene Bedeutung haben, unter reduzierenden Bedingungen umsetzt, oder

f) indem man Verbindungen der Formel IX

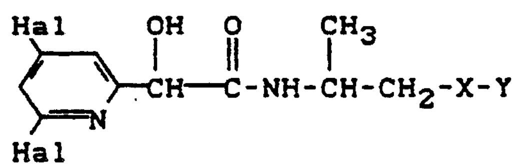

(IX)

in welcher Hal, X und Y die oben angegebene Bedeutung haben, reduziert.

3. Neue Verbindungen der Formel IX

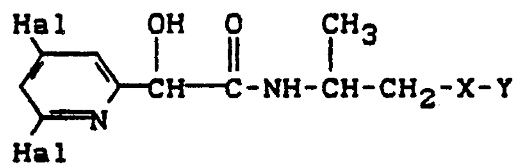

(IX)

in welcher Hal, X und Y die bei den Verbindungen der Formel I angegebenen Bedeutungen haben.

4. Verfahren zur Herstellung der Verbindungen der Formel IX, dadurch gekennzeichnet, daß man Verbindungen der Formel X

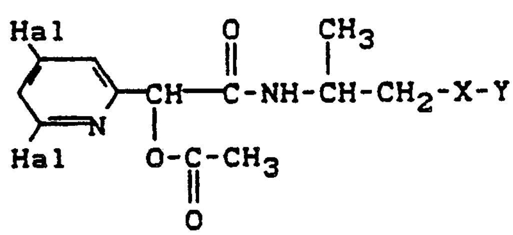

(X)

in welcher Hal, X und Y die oben angegebene Bedeutung haben, hydrolysiert.

5. Neue Verbindungen der Formel X

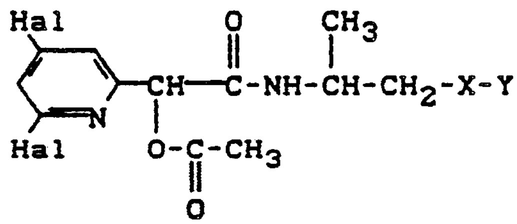

(X)

in welcher
Hal, X und Y die oben angegebene Bedeutung haben.

6. Verfahren zur Herstellung der Verbindungen der Formel X, dadurch gekennzeichnet, daß man Aldehyde der Formel XI

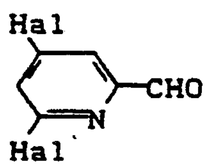

(XI)

in welcher
Hal für Halogen steht,
$R^1$, $R^2$, $R^3$ die oben angegebene Bedeutung haben,
mit Isonitrilen der Formel XII

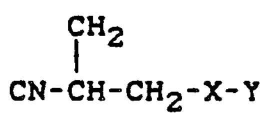

(XII)

in welcher
X und Y die oben angegebene Bedeutung haben,
in Gegenwart von Essigsäure umsetzt.

7. Neue Verbindungen der Formel II

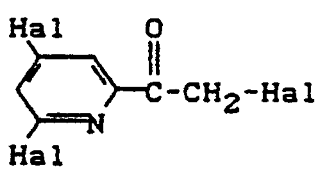

(II)

in welcher
Hal für Halogen steht.

8. Verfahren zur Herstellung der Verbindungen der Formel II, dadurch gekennzeichnet, daß man Acetylverbindungen der Formel XIII

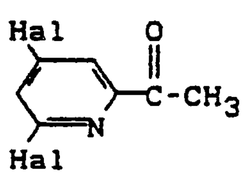

(XIII)

in welcher
Hal für Halogen steht,

a) mit elementarem Halogen gegebenenfalls in Gegenwart eines Katalysators umsetzt oder
b) mit Kupferhalogeniden der Formel

Cu $Hal_2$

c) oder mit anorganischen Halogeniden der Formel $SO_2Hal_2$

umsetzt.

9. Neue Pyridylacetylverbindungen der Formel XIII

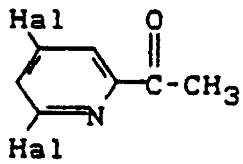

(XIII)

in welcher
Hal für Halogen steht.

10. Verfahren zur Herstellung der Pyridylacetylverbindungen der Formel XIII

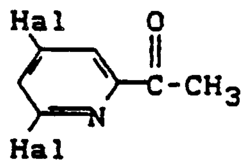

(XIII)

in welcher
Hal für Halogen steht,
dardurch gekennzeichnet, daß man 2,4-Dihalogen-6-pyridylcarbonsäure mit Methyllithium umsetzt.

11. Neue Verbindungen der Formel IV

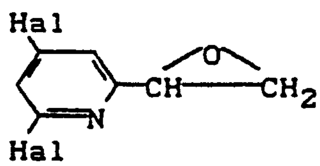

(IV)

in welcher
Hal für Halogen steht.

12. Verfahren zur Herstellung der Verbindungen der Formel IV

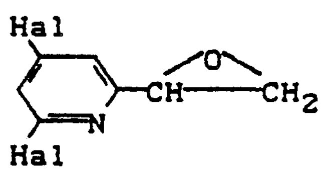

(IV)

in welcher
Hal für Halogen steht,
dadurch gekennzeichnet, daß man

a) Verbindungen der Formel V

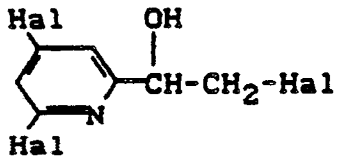

(V)

in welcher
Hal für Halogen steht,
mit Basen umsetzt, oder

b) Aldehyde der Formel XI

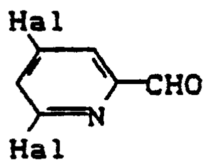

(XI)

in welcher
Hal für Halogen steht,
mit Methylgruppen übertragenden Reagenzien in Gegenwart von Basen unter den Bedingungen der Corey-Epoxidierung umsetzt.

13. Neue Verbindungen der Formel V

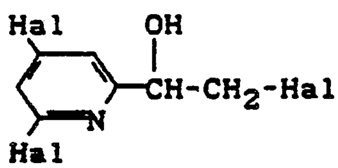

(V)

in welcher
Hal für Halogen steht.

14. Verfahren zur Herstellung der Verbindungen der Formel V

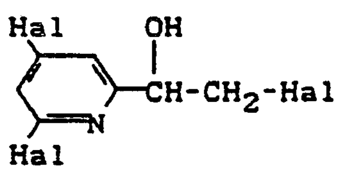

(V)

in welcher
Hal für Halogen steht,
dadurch gekennzeichnet, daß man Verbindungen der Formel II

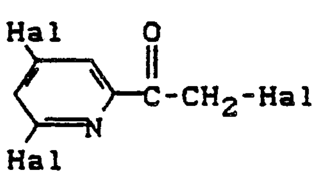

(II)

in welcher
Hal für Halogen steht,
reduziert.

15. Neue Verbindungen der Formel VI

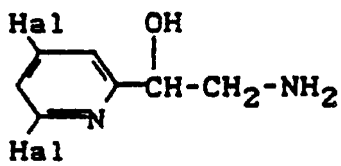

(VI)

in welcher
Hal für Halogen steht.

16. Verfahren zur Herstellung der Verbindungen der Formel VI

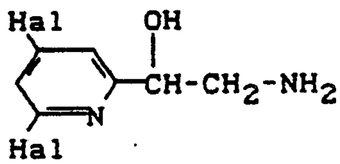

(VI)

in welcher
Hal für Halogen steht,
dadurch gekennzeichnet, daß man Cyanhydrine der Formel XIV

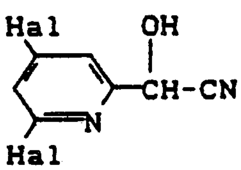

(XIV)

in welcher
Hal für Halogen steht,
reduziert.

17. Neue Verbindungen der Formel XIV

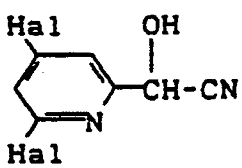

(XIV)

in welcher
Hal für Halogen steht.

18. Verfahren zur Herstellung der neuen Verbindungen der Formel XIV

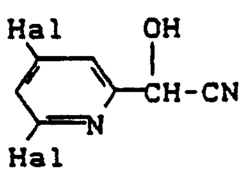

(XIV)

in welcher
Hal für Halogen steht,
dadurch gekennzeichnet, daß man Verbindungen der Formel XI

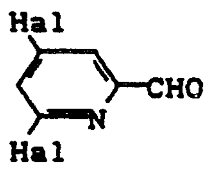

(XI)

in welcher
Hal für Halogen steht,
mit HCN oder Cyanidsalzen umsetzt.

19. Neue Verbindungen der Formel VIII

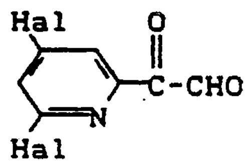

(VIII)

in welcher
Hal für Halogen steht.

20. Verfahren zur Herstellung der neuen Verbindungen der Formel VIII

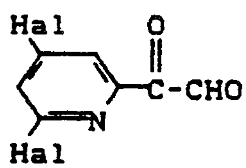

(VIII)

in welcher
Hal für Halogen steht,
dadurch gekennzeichnet, daß man Verbindungen der Formel V

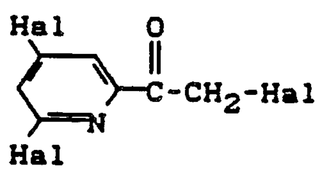

(V)

in welcher
Hal für Halogen steht,
oxidiert.

Die Verbindungen der Formel I können in Form ihrer sterischen und optischen Isomeren vorliegen und dabei zueinander enantiomere und/oder diastereomere Formen bilden.

Physiologisch verträgliche Salze der Verbindungen der Formel I können mit folgenden Säuren gebildet werden:
Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I
in welcher
Hal für Fluor, Chlor, Brom, insbesondere Chlor, steht,
X für eine direkte Bindung steht,
Y für Phenyl steht, das durch Hydroxy, Methoxy, Ethoxy, Hydroxyethoxy, Methoxyethoxy, Ethoxyethoxy, Methoxycarbonylmethoxy, Ethoxycarbonylethoxy, Phenylethylenoxy, Phenylpropylenoxy substituiert ist.

Besonders bevorzugt sind Verbindungen der Formel I, in welcher
Hal für Chlor steht,
X für eine direkte Bindung steht,
Y für Phenyl steht, das in 4-Stellung durch Hydroxy, Methoxy, Ethoxy, Hydroxyethoxy, Methoxyethoxy, Ethoxyethoxy, Methoxycarbonylmethoxy, Ethoxycarbonylethoxy substituiert ist.

Im einzelnen seien genannt:

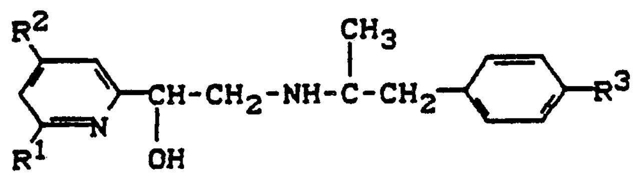

| $R^1$ | $R^2$ | $R^9$ |
|---|---|---|
| Cl | Cl | $OCH_2CH_2OH$ |
| Cl | Cl | $OCH_2COOCH_3$ |
| Cl | Cl | $OCH_2COOH$ |
| Cl | Cl | $COOC_2H_5$ |
| Cl | Br | $OCH_2CH_2NH_2$ |
| Br | Br | $COOCH_3$ |
| Cl | Br | $OCH_2COOCH_3$ |
| Br | Br | $OCH_2COOCH_3$ |
| Cl | Cl | $COOCH_3$ |
| Br | Br | $OCH_2COOH$ |

Bevorzugt seien die Salze mit Salzsäure, Schwefelsäure, Phosphorsäure, Oxalsäure, Maleinsäure, Fumarsäure, Malonsäure genannt.

Die Verbindungen der Formel I lassen sich nach den oben unter 2. angegebenen Verfahren a) bis f) herstellen.

Setzt man bei Verfahren 2a) als Halogenmethylketon der Formel II 2-Chloracetyl-4,6-dichlorpyridin und als Amin der Formel III 2-(4-Methoxycarbonylphenyl)isopropylamin ein, läßt sich Verfahren a) durch folgendes Reaktionsschema wiedergeben:

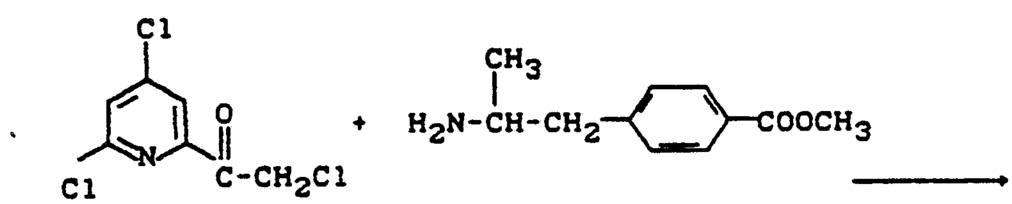

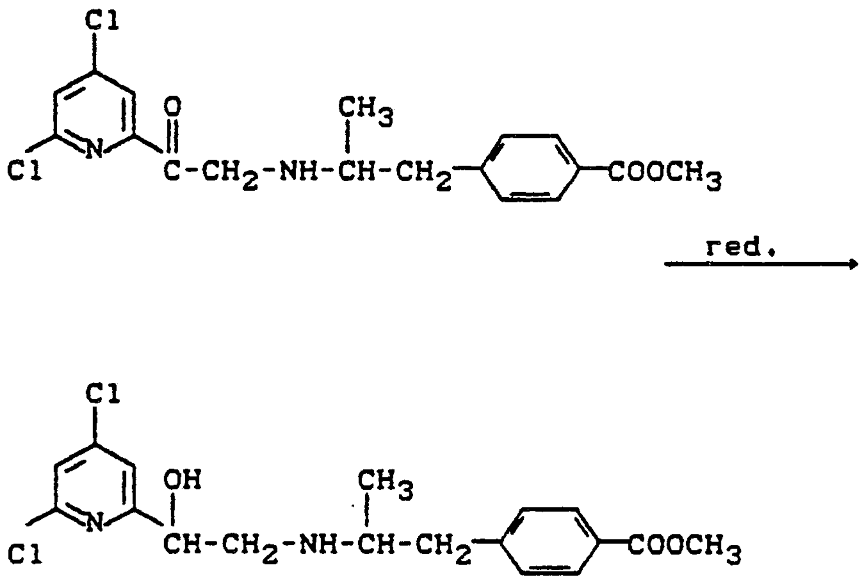


Die Verbindungen der Formel II werden nach dem unter (8) angegebenen Verfahren hergestellt, indem man in an sich bekannter Weise die entsprechenden acetylsubstituierten Heteroarylverbindungen mit elementarem Halogen, anorganischem Säurehalogenid oder mit Kupferhalogeniden umsetzt.

Die Substituenten Hal in Formel II besitzen bevorzugt die weiter oben bei den Verbindungen der Formel I angegebenen bevorzugten Bedeutungen. Im einzelnen seien folgende Verbindungen der Formel II genannt:

(2,4-Dichlor-6-pyridyl)-chlormethylketon,
(2,4-Dichlor-6-pyridyl)-brommethylketon,
(2,4-Dibrom-6-pyridyl)-brommethylketon,
(2,4-Difluor-6-pyridyl)-chlormethylketon.

Die Amine der Formel III sind bekannt (vgl. z.B. EP-OS 70 133) oder lassen sich analog zu bekannten Verfahren herstellen. Die Substituenten X und Y besitzen bevorzugt die weiter oben bei den Verbindungen der Formel I angegebenen bevorzugten Bedeutungen. Im einzelnen seien folgende Verbindungen der Formel III genannt:

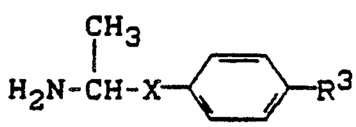

| X | $R^3$ |
|---|---|
| $-CH_2-$ | $OCH_2CH_2-O-C_2H_4$ |
| $-CH_2-$ | $OCH_2COOCH_3$ |
| $-CH_2-$ | $OCH_2COOH$ |
| $-CH_2-$ | $OCH_2CH_2OH$ |
| $-(CH_2)_2-$ | $OCH_2COOC_2H_5$ |

Als Reduktionsmittel zur Durchführung des Verfahrens 2a) seien folgende Reduktionsmittel genannt: $H_2$/Katalysator, als Katalysator seien beispielsweise genannt: $PtO_2$, Pd-Aktivkohle; komplexe Metallhydride wie z.B. $LiAlH_4$, $NaBH_4$, $NaBH_3CN$.

Besonders bevorzugt werden folgende Reduktionsmittel eingesetzt:

$NaBH_4$ und $NaBH_3CN$

Verfahren 2a) wird durchgeführt indem man Verbindungen II und III in einem Verdünnungsmittel in etwa äquimolaren Verhältnis zusammengibt.

Die Reaktion wird bei Temperaturen von -20° C bis +100° C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n- und i-Propanol.

Bevorzugt sind Alkohole, wobei ohne Isolierung der Zwischenstufen sofort die Reduktion durchgeführt werden kann.

Setzt man bei Verfahren 2b) als Epoxid der Formel IV 2,4-Dichlorpyridin-6-epoxid und als Amin der Formel III 3-(4-Ethoxycarbonylmethoxyphenyl)-2-aminopropan ein, läßt sich Verfahren 2b) durch folgendes Reaktionsschema wiedergeben:

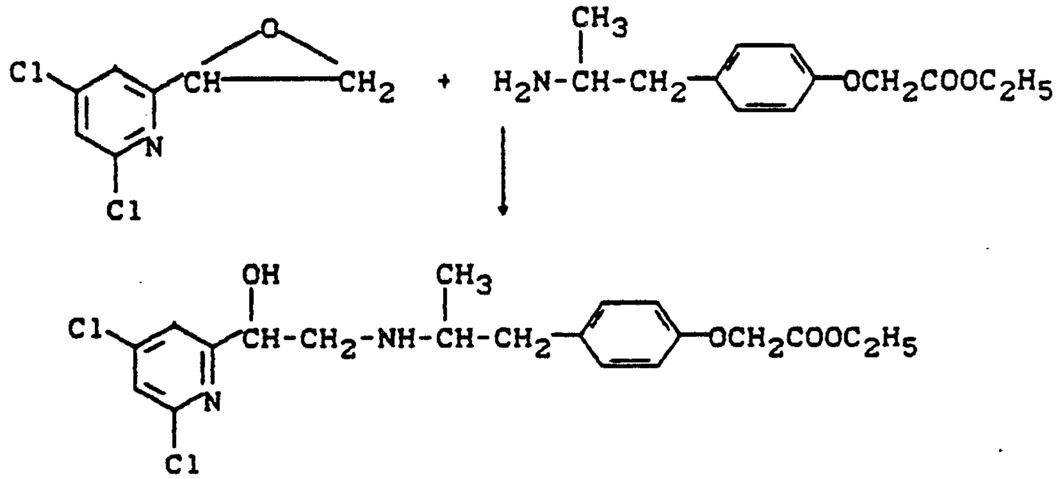

Epoxide der Formel IV werden nach dem unter (12) angegebenen Verfahren hergestellt.

Im einzelnen seien die folgenden Epoxide genannt:

2,4-Dichlor-pyridin-6-epoxid,

2,4-Dibrom-pyridin-6-epoxid.

Verfahren 2) wird durchgeführt indem man etwa äquimolare Mengen des Epoxids der Formel IV und des Amins der Formel III in einem Verdünnungsmittel umsetzt.

Im allgemeinen wird ein Überschuß an Amin (1-3 molar, bevorzugt 1-1,5 molar) bezogen auf das Epoxid der Formel IV verwendet.

Die Reaktion wird bei Temperaturen von +20 bis +150° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, ferner Nitrile wie Acetonitril und Benzonitril, Amide wie Dimethylformamid, Alkohole wie Methanol, Ethanol, n- und i-Propanol.

Bevorzugt sind Alkohole.

Setzt man bei Verfahren 2c) als β-Halogenmethylverbindung der Formel V 2,4-Dibrom-6-(1-hydroxy-2-chlorethyl)pyridin und als Amin der Formel III 2-(4-Methoxyphenyl)-1-methylethylamin ein, läßt sich Verfahren c) durch folgendes Formelschema wiedergeben:

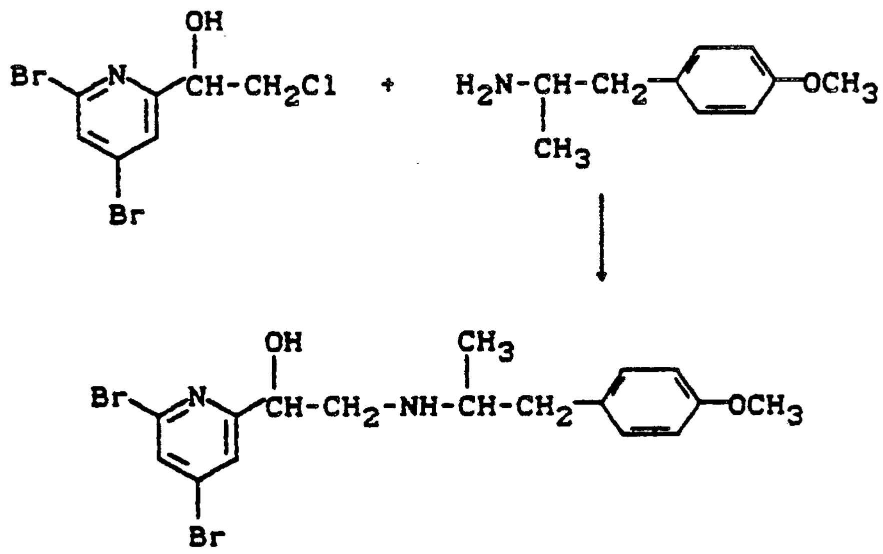

Die Herstellung der β-Halogenmethylverbindungen der Formel V erfolgt nach dem unter 14 beschriebenen Verfahren.

Im einzelnen seien folgende Verbindungen der Formel V genannt:
1-(2,4-Dibrom-6-pyridyl)-2-chlorethanol,
1-(2,4-Difluor-6-pyridyl)-2-chlorethanol,
1-(2,4-Dichlor-6-pyridyl)-2-chlorethanol,
1-(2,4-Dichlor-6-pyridyl)-2-bromethanol.

Verfahren 2c) wird durchgeführt indem man die beta-Halogenmethylverbindung der Formel V mit überschüssigem Amin der Formel III gegebenenfalls in Gegenwart eines Verdünnungsmittels umsetzt.

Die Reaktion wird bei Temperaturen von +20 bis +150 °C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und i-Propanol.

Bevorzugt eingesetzt werden Alkohole.

Setzt man bei Verfahren 2d) als Verbindung der Formel VI 2,4-Difluor-6-(1-hydroxy-2-aminoethyl)-pyridin und als Verbindung der Formel VII (3-Chlor-4-methoxyphenyl)aceton ein, läßt sich Verfahren d) durch folgendes Formelschema wiedergeben:

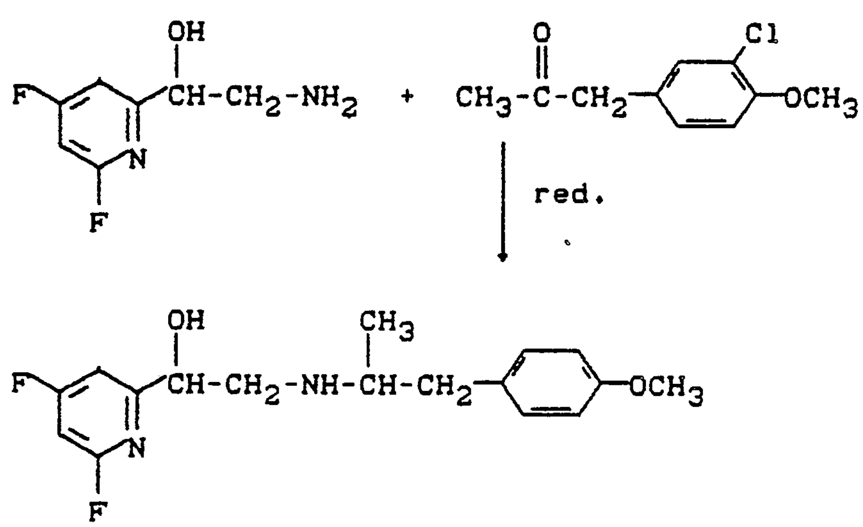


Die Verbindungen der Formel VI werden nach dem unter (16) angegebenen Verfahren hergestellt.

Im einzelnen seien folgende Verbindungen der Formel VI genannt:
1-(2,4-Dibrom-6-pyridyl)-2-aminoethanol,
1-(2,4-Difluor-6-pyridyl)-2-aminoethanol,
1-(2,4-Dichlor-3-amino-6-pyridyl)-2-aminoethanol,

Die Verbindungen der Formel VII sind bekannt (vgl. z.B. EP-OS 23 385, DE-OS 2 034 277, PCT-Appl. WO 84 00 956) oder lassen sich analog zu bekannten Verbindungen herstellen.

Im einzelnen seien folgende Verbindungen der Formel VII genannt:

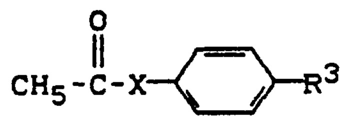

| X | $R^3$ |
|---|---|
| $-CH_2-$ | $-OCH_2CH_2-O-C_2H_4$ |
| $-CH_2-$ | $-COOCH_3$ |
| $-CH_2-$ | $-OCH_2COOCH_3$ |
| $-CH_2-$ | $-OCH_2CH_2OH$ |
| $-(CH_2)_2-$ | $-COOH$ |
| $-CH_2-$ | $-OCH_2COOH$ |

Verfahren 2d) wird durchgeführt, indem man etwa äquimolare Mengen der Verbindungen der Formeln VI und VII in einem Verdünnungsmittel vorlegt und die Mischung reduziert.

Die Reaktion wird bei Temperaturen von 0° C bis 150° C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol. Bevorzugt eingesetzt werden Alkohole.

Als Reduktionsmittel dienen:
$H_2$/Katalysator, als Katalysator sei beispielsweise $PtO_2$ genannt; komplexe Metallhydride wie z.B. $LiAlH_4$, $NaBH_4$, $NaBH_3CN$. Besonders bevorzugt werden eingesetzt: $NaBH_4$, $NaBH_3CN$.

Setzt man bei Verfahren 2e) als Verbindung der Formel VIII 2,4-Dibrom-6-pyridylglyoxal und als Amin der Formel III 2-(4-methoxyphenyl)-isopropylamin ein, läßt sich Verfahren e) durch folgendes Reaktionsschema wiedergeben:

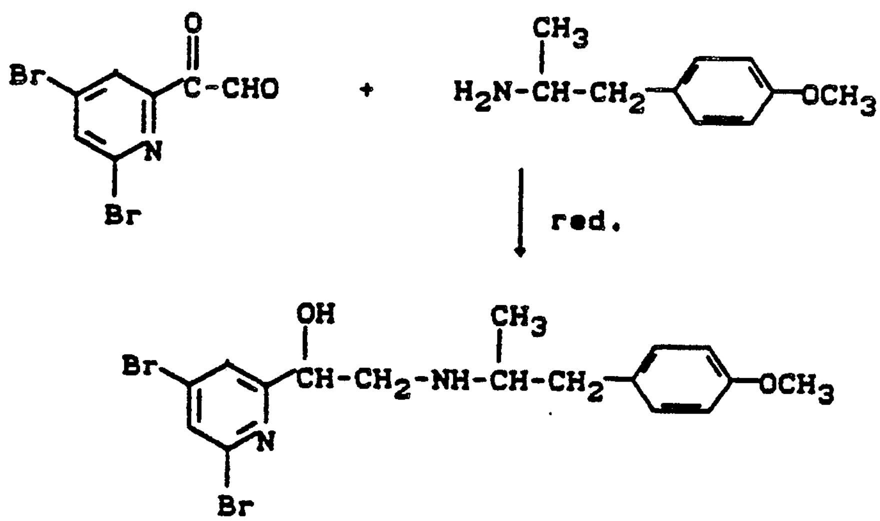


Die Herstellung der Verbindungen der Formel VIII erfolgt nach dem unter (20) angegebenen Verfahren. Verbindungen der Formel III sind bekannt (vgl. z.B. EP-OS 70 133) oder lassen sich analog zu bekannten Verbindungen herstellen.

Im einzelnen seien folgende Verbindungen der Formel VIII genannt:
2,4-Dibrom-6-pyridylglyoxal,
2,4-Difluor-6-pyridylglyoxal,
2,4-Dichlor-6-pyridylglyoxal.

Verfahren 2e) wird durchgeführt, indem man zur Verbindung der Formel VIII in einem Verdünnungsmittel etwa die äquivalente Menge des Amins der Formel III zusetzt und anschließend reduziert.

Die Reaktion wird bei Temperaturen von 0° C bis 100° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n- und i-Propanol.

Als Reduktionsmittel dienen
$H_2$/Katalysator; als Katalysator seien $PtO_2$ und Pd-Kohle genannt, ferner komplexe Metallhydride wie $LiAlH_4$ und $NaBH_4$.

Setzt man bei Verfahren 2f) als Verbindung der Formel X (2,4-Dichlor-6-pyridyl)hydroxyessigsäure-2(4-methoxyphenyl)isopropyl-amid ein, läßt sich das Verfahren durch folgendes Formelschema wiedergeben:

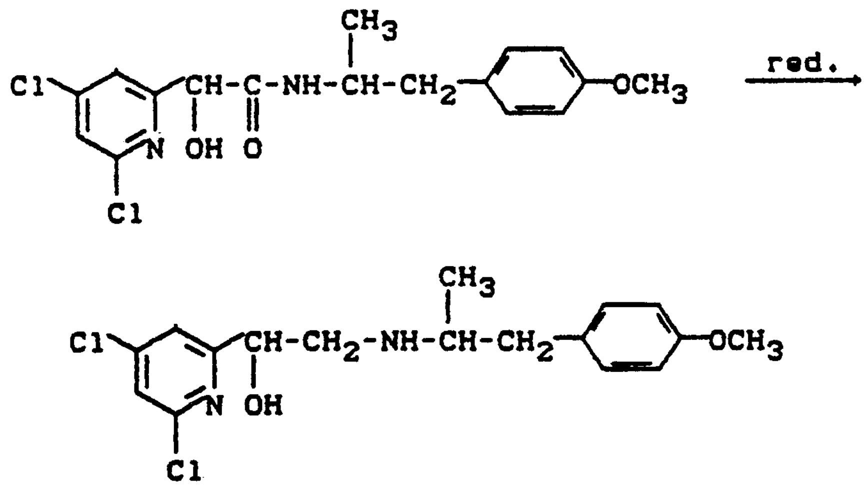


Die Verbindungen der Formel X sind neu. Ihre Herstellung wird unter (4) beschrieben. Im einzelnen seien folgende Verbindungen der Formel X genannt:

(2,4-Dichlor-6-pyridyl)hydroxyessigsäure-(3-(4-methoxycarbonylmethoxyphenyl)-2-propyl)amid
(2,4-Dichlor-6-pyridyl)hydroxyessigsäure-(3-(4-ethoxycarbonylphenyl)-2-propyl)amid
(2,4-Dibrom-6-pyridyl)hydroxyessigsäure-(3-(4-methoxycarbonylmethoxyphenyl)-2-propyl)amid
(2,4-Dichlor-6-pyridyl)hydroxyessigsäure-(3-(4-ethoxycarbonylmethoxyphenyl)-2-propyl)amid
(2,4-Dibrom-6-pyridyl)hydroxyessigsäure-(3-(4-methoxycarbonylphenyl)-2-propyl)amid
(3,4-Dibrom-6-pyridyl)hydroxyessigsäure-(3-(4-ethoxycarbonylmethoxyphenyl)-2-propyl)amid

Verfahren 2f) wird durchgeführt, indem man die Verbindung der Formel X in einem Verdünnungsmittel mit überschüssigem Reduktionsmittel umsetzt.

Die Reaktion wird bei Temperaturen von 0° C bis +150° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan.

Als Reduktionsmittel dienen komplexe Metallhydride wie $LiAlH_4$, Borane wie Diboran.

Verbindungen der Formel IX sind nach Verfahren 4 erhältlich.

Setzt man bei Verfahren 4) als Verbindung der Formel X (2,4-Dichlor-6-pyridyl)-acetoxyessigsäure-(2-(-4-ethoxyphenyl)ethylamid ein, läßt sich das Verfahren durch das folgende Schema darstellen:

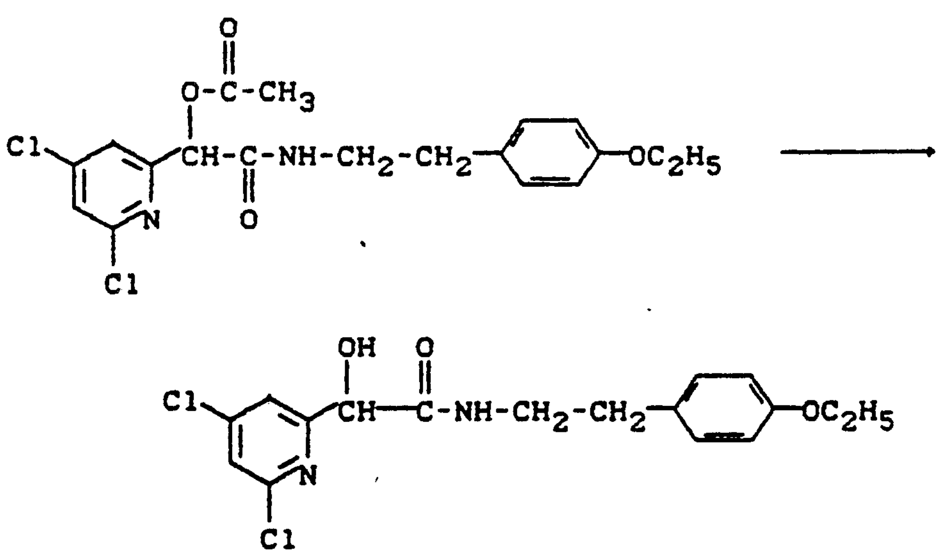

Die Verbindungen der Formel X sind neu. Ihre Herstellung erfolgt nach dem unter (6) angegebenen Verfahren. Im einzelnen seien folgende Verbindungen der Formel X genannt:

(2,4-Dichlor-6-pyridyl)acetoxyessigsäure-(3-(4-methoxycarbonylmethoxyphenyl)-2-propyl)amid
(2,4-Dichlor-6-pyridyl)acetoxyessigsäure-(3-(4-ethoxycarbonylphenyl)-2-propyl)amid
(2,4-Dibrom-6-pyridyl)acetoxyessigsäure-(3-(4-methoxycarbonylmethoxyphenyl)-2-propyl)amid
(2,4-Dichlor-6-pyridyl)acetoxyessigsäure-(3-(4-ethoxycarbonylmethoxyphenyl)-2-propyl)amid
(2,4-Dibrom-6-pyridyl)acetoxyessigsäure-(3-(4-methoxycarbonylphenyl)-2-propyl)amid
(2,4-Dibrom-6-pyridyl)acetoxyessigsäure-(3-(4-ethoxycarbonylmethoxyphenyl)-2-propyl)amid
(2,4-Difluor-6-pyridyl)acetoxyessigsäure-(3-(4-methoxycarbonylphenyl)-2-propyl)amid.

Zur Abspaltung der Acetylgruppe werden anorganische Säuren oder Laugen verwendet. Hierzu zählen Halogenwasserstoffsäuren wie Salzsäure; Schwefelsäure, Phosphorsäure, NaOH, KOH.

Das Verfahren wird durchgeführt, indem man die Verbindung X in einem Verdünnungsmittel als Lösungsvermittler mit überschüssiger wäßriger Lösung der anorganischen Säure oder Lauge behandelt.

Die Reaktion wird bei Temperaturen von +20 °C bis +150 °C durchgeführt.

Es wird bevorzugt bei Normaldruck gearbeitet.

Als Verdünnungsmittel können alle inerten organischen Lösungsmittel, die mit Wasser mischbar sind, verwendet werden. Hierzu zählen Ether wie Tetrahydrofuran, Dioxan; Nitrile, wie Acetonitril; Amide wie Dimethylformamid; Alkohole wie Methanol, Ethanol; Dimethylsulfoxid.

Verbindungen der Formel X sind nach Verfahren (6) erhältlich.

Setzt man bei Verfahren 6) als Verbindung der Formel XI 2,4-Difluor-pyridin-6-aldehyd und als Isonitril der Formel XIII 3-(4-Methoxyphenyl)-2-propylisonitril ein, läßt sich das Verfahren durch das folgende Schema wiedergeben:

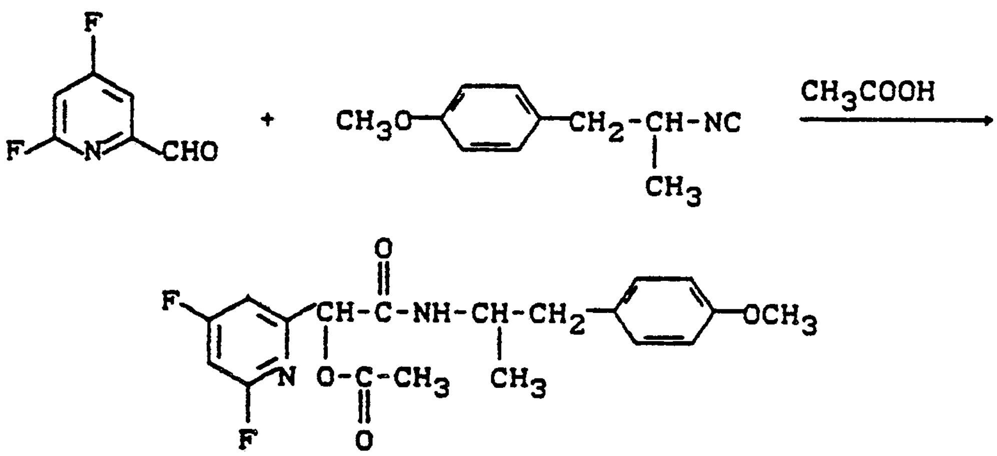


Aldehyde der Formel XI sind bekannt, z. B. aus J. Graf, J. Pr. Chem. (2) 134 (1932), S. 177, 180, oder lassen sich analog zu bekannten Verfahren herstellen. Im einzelnen seien die folgenden Verbindungen der Formel XI genannt:

2,4-Dichlorpyridin-6-aldehyd
2,4-Dibrompyridin-6-aldehyd
2,4-Difluorpyridin-6-aldehyd

Isonitrile der Formel XII sind bekannt (I. Ugi et al., Angew. Chem. 77 (1965), 492) oder lassen sich analog zu bekannten Verfahren herstellen. Im einzelnen seien folgende Verbindungen der Formel XII genannt:

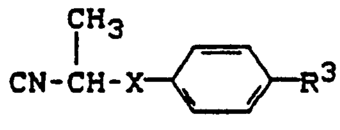

| X | $R^3$ |
|---|---|
| $-CH_2-$ | $-O-CH_2CH_2-O-C_2H_4$ |
| $-CH_2-$ | $-COOCH_3$ |
| $-CH_2-$ | $-OCH_2COOCH_3$ |
| $-CH_2-$ | $-OCH_3$ |
| $-(CH_2)_2-$ | $-OCH_2CH_2OH$ |

Das Verfahren wird durchgeführt, indem man die Verbindung XI mit der doppelt molaren Menge des Isonitrils der Formel XII und Essigsäure in einem Verdünnungsmittel umsetzt.

Die Reaktion wird bei Temperaturen von 0° C bis +150° C durchgeführt.

Es wird bevorzugt bei Normaldruck gearbeitet.

Als Verdünnungsmittel dienen alle inerten organischen Lösungsmittel. Hierzu gehören insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril und Benzonitril.

Verbindungen der Formel II sind nach den Verfahren 8 a, b erhältlich.

Setzt man bei Verfahren 8 a) als Verbindung der Formel XIII 2,4-Dichlor-6-acetylpyridin und als Halogen Hal Brom ein, läßt sich die Reaktion durch folgendes Schema darstellen:

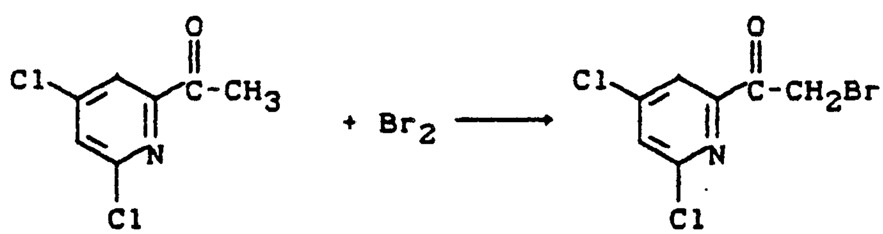


Die Verbindungen der Formel XIII sind neu. Ihre Herstellung erfolgt nach dem unter (10) angegebenen Verfahren.

Im einzelnen seien folgende Verbindungen der Formel XIII genannt:
2,4-Dichlor-6-acetylpyridin,
2,4-Dibrom-6-acetylpyridin,

Verfahren 8 a) wird durchgeführt, indem man zu Verbindung XIII in einem Verdünnungsmittel die äquivalente Menge Halogen, eventuell in einem Verdünnungsmittel gelöst, zugibt.

Die Reaktion wird bei +20° C bis +150° C durchgeführt, bevorzugt bei der Siedetemperatur des verwendeten Verdünnungsmittels.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Setzt man bei Verfahren 8 b) als Verbindung der Formel XIII 2,4-Dibrom-6-acetylpyridin und als Verbindung der Formel $CuHal_2$ Kupfer(II)bromid ein, läßt sich die Reaktion durch folgendes Schema darstellen:

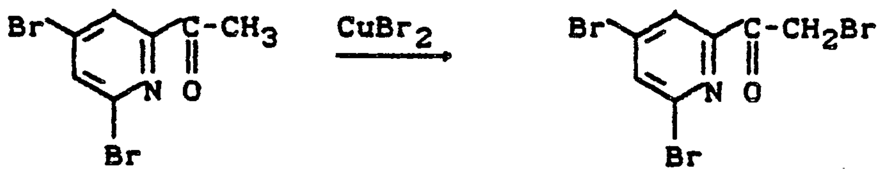


Verfahren 8 b) wird durchgeführt, indem man äquivalente Mengen der Verbindung der Formel XI und der Verbindung $CuHal_2$ im Verdünnungsmittel 1-24 h, bevorzugt 6-12 h unter Rückfluß erhitzt.

Die Reaktion wird ansonsten wie bei Verfahren 8 a) beschrieben durchgeführt.

Setzt man bei Verfahren 8c) als Verbindung der Formel (XIII) 2,4-Dichlor-6-acetyl-Pyridin und als anorganisches Säurehalogenid $SO_2Cl_2$ ein, so läßt sich die Reaktion durch folgendes Schema darstellen:

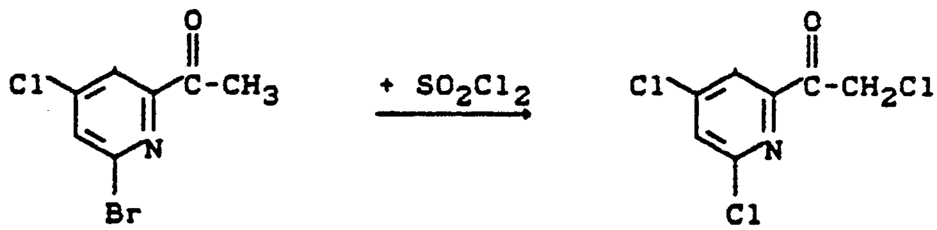


Die Reaktion wird ansonsten wie bei Verfahren 8 a) beschrieben durchgeführt.

Pyridylacetylverbindungen der Formel XIII sind nach dem bei (10) angegebenen Verfahren erhältlich. Verfahren (10) kann durchgeführt werden, wie in US-P 4 358 455 beschrieben. Die als Ausgangsprodukt eingesetzten 2,4-Dihalogen-6-pyridyl-carbonsäuren sind bekannt oder können analog zu bekannten Verfahren hergestellt werden (Graf, J. Pr. Chem. (2) 134 (1932) S. 177 - 187).

Epoxide der Formel IV sind nach den bei (12) angegebenen Verfahren erhältlich.

Verfahren 12 a) wird durchgeführt, indem man eine Verbindung der Formel V in einem Verdünnungsmittel mit der 2-5 molaren, bevorzugt 2-4 molaren Menge einer Base umsetzt. Setzt man als Verbindung der Formel V 1-(2,4-Dichlor-6-pyridyl)-2-bromethanol und als Base NaOH ein, läßt sich die Reaktion durch folgendes Schema darstellen:

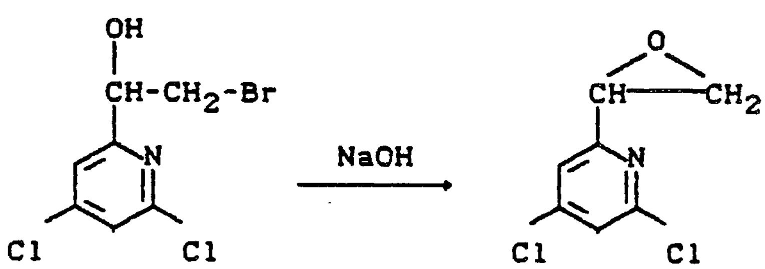


Im einzelnen seien folgende Verbindungen der Formel V genannt:
1-(2,4-Dichlor-6-pyridyl)-2-bromethanol,
1-(2,4-Dichlor-6-pyridyl)-2-chlorethanol.

Als Basen seien genannt: Alkali- und Erdalkalihydroxide wie Natriumhydroxid, Kaliumhydroxid; -carbonate und -hydrogencarbonate wie Natriumcarbonat, Natriumhydrogencarbonat, Bariumcarbonat; -alkoholate wie Natriummethylat und Natriumethylat.

Als Verdünnungsmittel seien genannt Alkohole, wie Methanol, Ethanol, Wasser, sowie Mischungen von Alkoholen mit Wasser.

Die Reaktion wird bei Temperaturen von 0 °C bis +100 °C durchgeführt, es wird bevorzugt unter Normaldruck gearbeitet.

Setzt man bei Verfahren 12 b) als Aldehyd der Formel XI 2,4-Dibrompyridin-6-aldehyd und als methylengruppenübertragendes Reagenz Trimethylsulfoniumiodid sowie als Base Natriumhydrid ein, läßt sich die Umsetzung durch folgendes Schema darstellen:

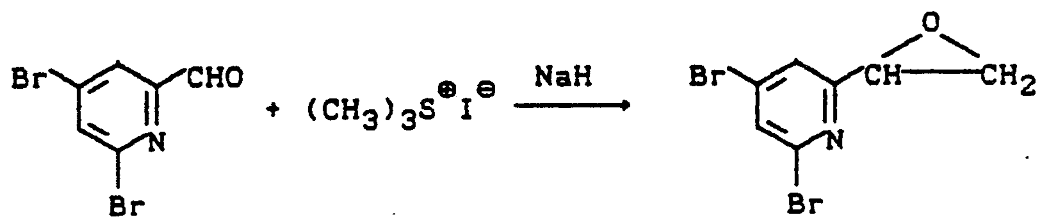


Im einzelnen seien folgende Verbindungen der Formel XII genannt:
2,4-Dichlorpyridin-6-aldehyd,
2,4-Dibrompyridin-6-aldehyd,
2,4-Difluorpyridin-6-aldehyd.

Als methylengruppenübertragende Reagentien seien genannt:
Trimethylsulfoniumhalogenide wie Trimethylsulfoniumchlorid, -bromid und -iodid,
Trimethylsulfoxoniumhalogenide wie Trimethylsulfoxoniumchlorid, -bromid und -iodid.

Als Basen werden verwendet: Alkali- und Erdalkalihydride wie Natriumhydrid, Alkali- und Erdalkalialkoholate wie Kalium-tert.-butylat.

Verfahren 12 b) wird durchgeführt, indem man 1,1-Äquivalente der Base in Dimethylsulfoxid vorlegt, dann das methylengruppenübertragende Agens (1,1 Äquivalente) zufügt und zuletzt 1 Äquivalent der Verbindung der Formel XII zugibt.

Die Reaktion wird bei Temperaturen von 0° C bis 100° C, bevorzugt bei 50-70° C durchgeführt, es wird bevorzugt bei Normaldruck gearbeitet.

Als Verdünnungsmittel werden Dimethylsulfoxid oder Mischungen von Dimethylsulfoxid mit inerten organischen Lösungsmitteln eingesetzt.

Als inerte organische Lösungsmittel seien genannt: Ether wie Diethylether, Tetrahydrofuran, Dioxan.

Verbindungen der Formel V sind nach Verfahren (14) erhältlich.

Setzt man bei Verfahren (14) als Verbindung der Formel II (2,4-Dichlor-6-pyridyl)chlormethylketon ein, läßt sich Verfahren (14) durch das folgende Reaktionsschema darstellen:

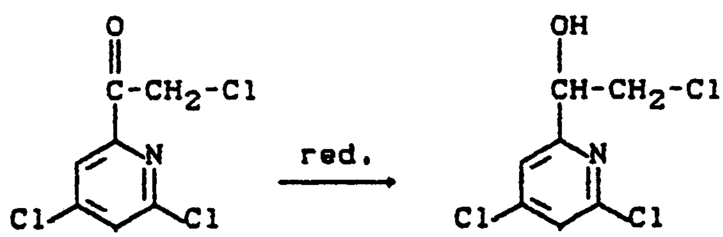


Die Verbindungen der Formel II sind neu und werden nach den unter (8) genannten Verfahren erhalten. Bevorzugt werden Verbindungen der Formel II eingesetzt, die im Verfahren (2 a) genannt sind.

Als Reduktionsmittel zur Durchführung des Verfahrens (14) seien genannt:
$H_2$/Katalysator, als Katalysatoren seien genannt: $PtO_2$, Pd/Kohle; komplexe Metallhydride, wie z.B. $LiAlH_4$, $NaBH_4$, $NaBH_3CN$. Bevorzugt werden $NaBH_4$ und $NaBH_3CN$ eingesetzt.

Verfahren (14) wird durchgeführt, indem die Verbindung II in einem Verdünnungsmittel mit dem Reduktionsmittel umsetzt.

Die Reaktion wird bei Temperaturen von -20° C bis +100° C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril; Alkohole wie Methanol, Ethanol, n- und i-Propanol. Bevorzugt werden Alkohole eingesetzt.

Verbindungen der Formel VI sind nach Verfahren (16) erhältlich.

Setzt man bei Verfahren (16) als Verbindung der Formel XIV 2,4-Dichlor-6-pyridylcyanhydrin ein, läßt sich das Verfahren durch das folgende Schema darstellen:

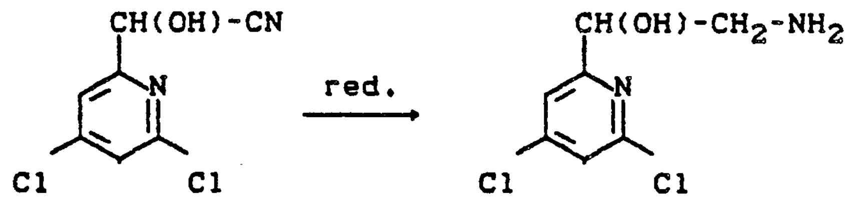


Die Verbindungen der Formel XIV sind neu. Ihre Herstellung wird weiter unten beschrieben. Im einzelnen seien folgende Verbindungen der Formel XIV genannt:
(2,4-Dichlor-6-pyridyl)-cyanhydrin
(2,4-Dibrom-6-pyridyl)-cyanhydrin.

Das Verfahren wird durchgeführt, indem man die Verbindung XIV in einem Verdünnungsmittel reduziert.

Die Reaktion wird bei Temperaturen von 0° C bis 150° C durchgeführt.

Es wird bei Normaldruck oder bei erhöhtem Druck gearbeitet.

Als Verdünnungsmittel dienen, abhängig von Reduktionsmittel, Wasser oder organische Lösungsmittel oder Mischungen hiervon. Zu den organischen Lösungsmitteln gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:
$H_2$/Katalysator, als Katalysator sei beispielsweise $PtO_2$ genannt; Alkali- und Erdalkaliamalgame wie z. B. Natriumamalgam; unedle Metalle in Gegenwart von Salzsäure wie z. B. Zink/Salzsäure; komplexe Metallhydride wie z. B. $LiHlH_4$; Borane wie z. B. Diboran.

Verbindungen der Formel XIV sind nach Verfahren (18) erhältlich.

Setzt man zur Durchführung von Verfahren (18) als Verbindung der Formel XI 2,4-Difluorpyridin-6-aldehyd ein, läßt sich das Verfahren durch das folgende Schema wiedergeben:

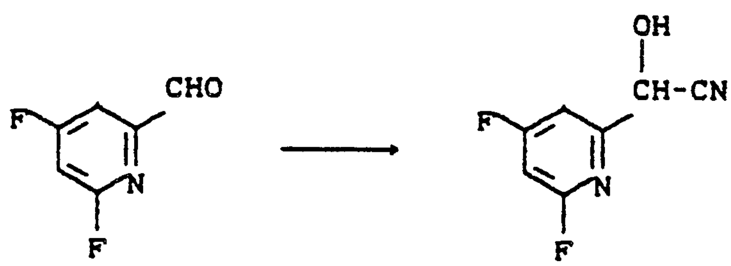

Aldehyde der Formel XI sind bekannt (R. Graf, J. Pr. Chem. 134 (1932) S. 177 - 187).

Im einzelnen seien folgende Verbindungen der Formel XI genannt:
2,4-Dichlorpyridin-6-aldehyd
2,4-Dibrompyridin-6-aldehyd.

Das Verfahren wird durchgeführt, indem man in literaturbekannter Weise die Aldehyde der Formel XI bzw. deren Hydrogensulfitadditionsprodukte mit Cyanwasserstoff oder ihren Salzen oder niederaliphatischen Ketoncyanhydrinen umsetzt (P.Kurtz, in Houben-Weyl, Bd. VIII, S. 274 ff).

Verbindungen der Formel VIII sind nach Verfahren (20) erhältlich.

Setzt man bei Verfahren (20) als Halogenmethylketon der Formel V (2,4-Dichlor-6-pyridyl)-brommethylketon ein, läßt sich das Verfahren durch das folgende Schema darstellen:

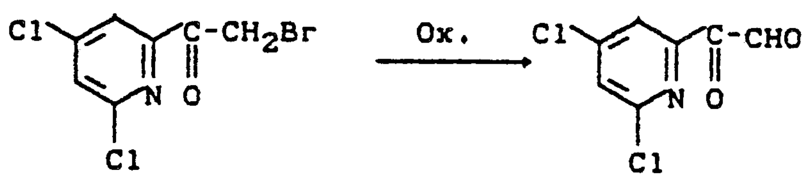


Als Halogenmethylketone der Formel V werden bevorzugt die bei Verfahren (2) genannten Verbindungen eingesetzt.

Das Verfahren wird durchgeführt, indem man die Verbindungen der Formel V, gegebenenfalls in Gegenwart eines Verdünnungsmittels, oxidiert.

Die Reaktion wird bei Temperaturen von +20°C bis +100°C durchgeführt.

Es wird bevorzugt bei Normaldruck gearbeitet.

Als Oxidationsmittel wird bevorzugt Dimethylsulfoxid verwendet (N. Kornblum et. al., JACS **79**, 6562 (1957).

Wird die Umsetzung in Gegenwart eines Verdünnungsmittels durchgeführt, können alle inerten organischen Lösungsmittel verwendet werden. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril. Bevorzugt wird in Dimethylsulfoxid ohne ein weiteres Lösungsmittel gearbeitet.

Die Wirkstoffe werden als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet. Sie dienen auch zur Behandlung der Adipositas bei Mensch und Tier.

Zu den Nutz- und Zuchttieren zählen Säugetiere wie z. B. Rinder, Schweine, Pferde, Schafe, Ziegen, Kaninchen, Hasen, Damwild, Pelztiere wie Nerze, Chinchilla, Geflügel wie z. B. Hühner, Gänse, Enten, Truthühner, Tauben, Fische wie z. B. Karpfen, Forellen, Lachse, Aale, Schleien, Hechte, Reptilien wie z. B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z.B. Goldfische.

Die Wirkstoffe werden unabhängig vom Geschlecht der Tiere während allen Wachstums- und Leistungsphasen der Tiere eingesetzt.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Körpergewicht, dem Geschlecht, dem Verfettungsgrad, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Ernährungs- und Gesundheitszustand der Tiere ab.

Die Verabreichung erfolgt oral oder parenteral in dafür geeigneten Formulierungen oder in reiner Form. Orale Formulierungen sind Pulver, Tabletten, Granulate, Doenche, Boli sowie Futtermittel, Prämixe für Futtermittel, Formulierungen zur Verabreichung über Trinkwasser.

Parenterale Formulierungen sind z.B. Injektionslösungen und Implantate.

Die oralen Formulierungen enthalten den Wirkstoff in Konzentrationen von 0,01 ppm - 100 %, bevorzugt von 0,01 ppm - 1 %.

Die Wirkstoffe können einmalig verabreicht werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

Die Wirkstoffe können in den Formulierungen allein oder in Mischung mit anderen Wirkstoffen, mineralischen Futtermitteln, Spurenelement-Verbindungen, Vitaminen, Stickstoff liefernden Nicht-Protein-Verbindungen, Farbstoffen, Antioxidantien, Aromastoffen, Emulgatoren, Fließhilfsstoffen, Konservierungs- und Preßhilfsstoffen vorliegen.

Andere Wirkstoffe sind:

z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid.

Vitamine sind z.B. Vitamin A, Vitamin $D_3$, Vitamin E, B-Vitamine, Vitamin C.

Stickstoff-liefernde Nicht-Protein-Verbindungen sind z.B. Biuret, Harnstoff.

Farbstoffe sind z.B. Carotinoide wie Citranaxanthin, Zeaxanthin, Capsanthin.

Antioxidantien sind z.B. Ethoxyquin, Butylhydroxy-Toluol.

Aromastoffe sind z.B. Vanillin.

Emulgatoren sind z.B. Ester der Milchsäure, Lecithin.
Fließhilfsstoffe sind z.B. Natriumstearat, Calciumstearat.
Konservierungsstoffe sind z.B. Zitronensäure, Propionsäure.
Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milch produkte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das 10 ppm erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g des unten angegebenen Wirkstoff-Prämix ergeben nach sorgfältigem Mischen 1 kg Futter mit 10 ppm Wirkstoffgehalt.

In einem kg Vitamin-Mineral-Mischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das 8 ppm erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter mit 8 ppm Wirkstoffgehalt.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

## Beispiel A

### Ratten-Fütterungsversuch

Weibliche Laborratten 150-165 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt und die relative Gewichtszunahme im Vergleich zur unbehandelten Kontrolle errechnet. Bei Versuchsende wird die Menge an perirenalem Fettgewebe bestimmt und im Vergleich zur unbehandelten Kontrolle berechnet.

Es werden die aus der Tabelle ersichtlichen Ergebnisse erhalten:

Tabelle 1

| Ratten Fütterungsversuch | | | |
|---|---|---|---|
| Wirkstoff Beispiel Nr. | Wirkstoff Aufwand ppm | Körpergewichtszunahme (Kontrolle = 00) | Menge an perirenalem Fettgewebe (Kontrolle = 100) |
| 1 | 25 | 188 | 69 |

Beispiel B

Pharmakologischer Versuch mit Beagle-Hunden zur Beurteilung der Verträglichkeit (Steigerung der Herzfrequenz)

Zur Beurteilung der Effekte der erfindungsgemäßen Wirkstoffe auf die mögliche Steigerung der Herzfrequenz werden Experimente mit wachen weiblichen Beagle-Hunden (10 - 15 kg Körpergewicht) durchgeführt. Der Behandlungsgruppe (3 Tiere) werden die Wirkstoffe unmittelbar nach der Morgenfütterung über eine Gelatine-Kapsel in der gewünschten Menge oral verabreicht. Die Negativ-Kontrollgruppe (3 Tiere) erhält ein Placebo. Die Herzfrequenz der Hunde wird 50, 90 und 150 Minuten nach der Substanzapplikation bestimmt und im Vergleich zur unbehandelten Kontrollgruppe berechnet.

Es werden die in der Tabelle ersichtlichen Ergebnisse erhalten.

Tabelle 2

| Pharmakologischer Versuch (Steigerung der Herzfrequenz) bei Beagle-Hunden | | | | |
|---|---|---|---|---|
| Wirkstoff Beispiel Nr. | Wirkstoff-aufwand (mg/kg KG) | Herzfrequenz (Schläge/Minute) Minuten nach Substanzapplikation | | |
| | | 50 | 90 | 150 |
| Unbeh. Kontrolle | 0 | 72 | 72 | 63 |
| Bsp.1 | 0,075 | 113 | 113 | 80 |

Herstellungsbeispiele

Beispiel 1

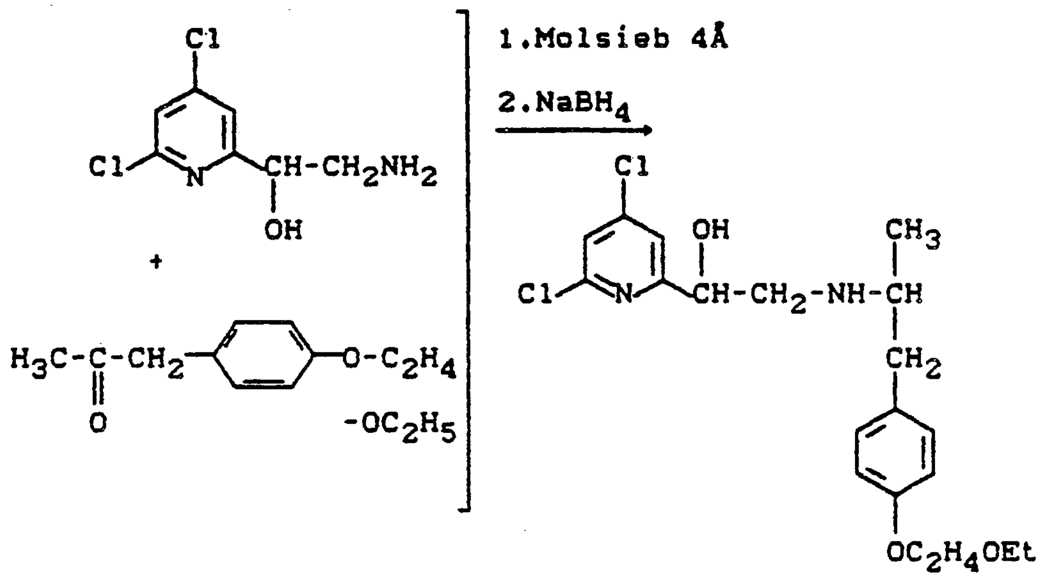


(RS)-2,4-Dichlor-α-[[[4-(2-ethoxyethoxy)phenethyl]amino]methyl]-6-pyridinmethanol

500 mg (2,42 mmol) 2-Amino-1-(2,4-dichlor-6-pyridyl)ethanol, 536 mg (2,42 mmol) 4-(2-Ethoxyethoxy)-phenylaceton und 4,6 g 3Å Molsieb werden in 100 ml trockenem Ethanol 48 Stunden bei Raumtemperatur gerührt. Anschließend wird auf 0 °C gekühlt und 389 mg (11 mmol) $NaBH_4$ zugegeben. Es wird erst 30 Minuten bei 0 °C, dann eine Stunde bei Raumtemperatur gerührt. Zur Aufarbeitung wird filtriert und das Filtrat im Vakuum eingedampft.

Der Rückstand wird in 100 ml Wasser suspendiert, mit konzentrierter HCl-Lösung angesäuert und dreimal mit $CH_2Cl_2$ extrahiert. Der Extrakt wird mit verdünnter NaOH-Lösung gewaschen, mit $Na_2SO_4$ getrocknet und eingedampft. Zur Reinigung wird über Kieselgel mit Essigester/Methanol chromatographiert. Ausbeute: 690 mg farbloses Öl, Diastereomerengemisch $^1$H-NMR($CDCl_3$) : 6,8 - 7,5 ppm (m, 6H, Harom), 4,6 ppm (m, 1H, >CH -OH), 4,1 ppm (m, 2H, -$CH_2$-O-), 3,8 ppm (m, 2H, -$CH_2$-O-), 3,6 ppm (δ, 2H, -O-$CH_2$-$CH_3$), 3,05 - 3,2 ppm (m, 1H, -CH-NH-), 2,7 - 2,9 ppm (m, 2H, -$CH_2$-NH-), 2,6 ppm (d, 2H, Ar-$CH_2$-CH), 1,25 ppm (t, 3H, $CH_2$-$CH_3$), 1,05 ppm (d, 3H, CH-$CH_3$).

4-(2-Ethoxyethoxy)phenylaceton wurde gemäß dem in DE-OS 2 034 277 beschriebenen Verfahren hergestellt.

Analog wurden hergestellt:

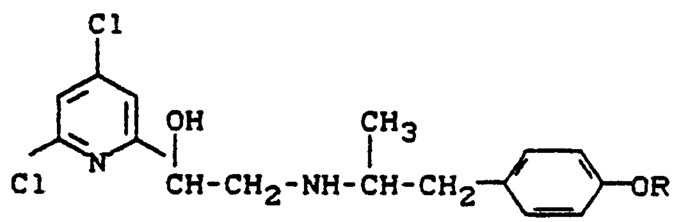

Beispiel 2

R = -H: Ausbeute: 413 mg farbloses Öl, Diastereomerengemisch
$^1$H-NMR($CDCl_3$): 6,8 - 7,5 ppm (m, 6H, Harom), 4,6 ppm (m, 1H, >CH -OH), 3,0 - 3,2 ppm (m, 1H, -CH-NH-), 2,7 - 2,9 ppm (m, 2H, -$CH_2$-NH-), 2,6 ppm (d, 2H, Ar-$CH_2$-CH), 1,05 ppm (d, 4H, CH-$CH_3$).

Beispiel 3

R = $-CH_2-CH_2OCH_3$
Ausbeute: 800 mg farbloses Öl, Diastereomerengemisch $^1H$-NMR($CDl_3$) : 6,8 - 7,5 ppm (m, 6H, Harom), 4,6 ppm (m, 1H, >CH -OH), 4,1 ppm (m, 2H, $-CH_2-O-$), 3,75 ppm (m, 2H, $-CH_2-O-$), 3,45 ppm (s, 3H, $-O-CH_3$), 3,05 - 3,2 ppm (m, 1H, -CH-NH-), 2,7 - 2,9 ppm (m, 2H, $-CH_2-NH-$), 2,6 ppm (d, 2H, $Ar-CH_2-CH$), 1,05 ppm (d, 3H, $-CHCH_3$)

Beispiel 4

R = $-CH_3$
Ausbeute: 760 mg farbloses Öl, Diastereomerengemisch $^1H$-NMR($CDl_3$) : 6,8 - 7,5 ppm (m, 6H, Harom), 4,6 ppm (m, 1H, >CH -OH), 3,8 ppm (s, 3H, $-OCH_3$), 3,05 - 3,2 ppm (m, 1H, -CH-NH-), 2,7 - 2,9 ppm (m, 2H, $-CH_2-NH-$), 2,6 ppm (d, 2H, $Ar-CH_2-CH$), 1,05 ppm (d, 3H, $CH-CH_3$)

Beispiel 5

R = $-CH_2-CH_2OH$
Ausbeute: 700 mg farbloses Öl, Diastereomerengemisch $^1H$-NMR($CDl_3$) : 6,8 - 7,5 ppm (m, 6H, Harom), 4,6 ppm (m, 1H, C -OH), 4,1 ppm (m, 2H, $-CH_2-O-$), 3,95 ppm (m, 2H, $-CH_2-O-$), 3,05 - 3,2 ppm (m, 1H, -CH-NH-), 2,7 - 2,9 ppm (m, 2H, $-CH_2-NH-$), 2,6 ppm (d, 2H, $Ar-CH_2-CH$), 1,05 ppm (d, 3H, $-CH-CH_3$)

Herstellung der Ausgangsverbindungen

Beispiel 6

Beispiel für Verfahren 16

1-(2,4-Dichlor-6-pyridyl)-2-aminoethanol

Zu 150 ml einer siedenden 1 M Lösung von Boran in THF wird die Lösung von 5,4 g (26,6 mmol) (2,4-Dichlor-6-pyridyl)-cyanhydrin in 60 ml THF getropft. Nach 10 Minuten wird mit 15 ml konzentrierter Salzsäure angesäuert, 15 Minuten gerührt, dann mit verdünnter Natronlauge auf pH 5 gestellt und das THF abgedampft. Nach Verdünnen mit Wasser wird zweimal mit Essigester gewaschen, mit verdünnter Lauge auf pH 11 gestellt und mit Essigester extrahiert. Nach Trocknen und Eindampfen erhält man 3,8 g (70 % d. Th.) der Titelverbindung, Fp. 117 - 118° C.

Beispiel 7

Beispiel für Verfahren 18

(2,4-Dichlor-6-pyridyl)-cyanhydrin

Zur Lösung von 4,8 g (27 mmol) 2,4-Dichlorpyridin-6-aldehyd in 50 ml Ether werden 9,8 g 40 %ige wässrige $NaHSO_3$-Lösung gegeben. Man verdünnt mit 200 ml Wasser und 200 ml Ether, kühlt auf 10° C und gibt 3,7 g NaCN zu. Der Ansatz wird 5 Minuten nachgerührt, die Etherphase wird abgetrennt, über $Na_2SO_4$ getrocknet und eingedampft. Der Rückstand kristallisiert an der Ölpumpe durch. Farblose Kristalle, Ausbeute 5,4 g (99 % d. Th.), Fp. 56° C.

**Ansprüche**

1. 2,4-Dihalogen-6-pyridyl-ethanolamine der Formel I

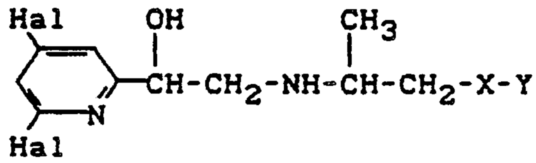 (I)

in welcher
Hal gleich oder verschieden für Fluor, Chlor, Brom stehen,
X für eine direkte Bindung oder $-CH_2-$ steht,
Y für Phenyl steht das durch OH, $C_{1-4}$-Alkoxy, $C_{1-4}$-Alkoxy-$C_{1-4}$-alkoxy, Carboxyl, Aminocarbonyl, $C_{1-4}$-Alkoxycarbonyl, Hydroxy-$C_{1-4}$-alkoxy, $C_{1-4}$-Alkoxycarbonyl-$C_{1-4}$-alkoxy, Phenyl-$C_{1-4}$-alkoxy substituiert ist sowie ihre physiologisch verträglichen Salze und N-Oxide.

2. Verfahren zur Herstellung der Verbindungen der Formel I

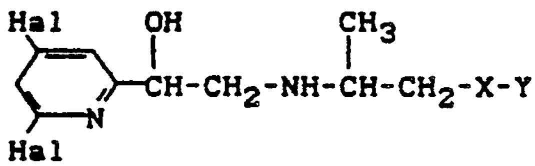 (I)

in welcher
Hal gleich oder verschieden für Fluor, Chlor, Brom stehen,
X für eine direkte Bindung oder $-CH_2-$ steht,
Y für Phenyl steht das durch OH, $C_{1-4}$-Alkoxy, $C_{1-4}$-Alkoxy-$C_{1-4}$-alkoxy, Carboxyl, Aminocarbonyl, $C_{1-4}$-Alkoxycarbonyl, Hydroxy-$C_{1-4}$-alkoxy, $C_{1-4}$-Alkoxycarbonyl-$C_{1-4}$-alkoxy, Phenyl-$C_{1-4}$-alkoxy substituiert ist

a) indem man Halogenmethylketone der Formel II

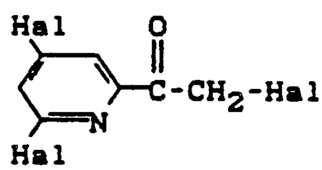 (II)

in welcher
Hal für Halogen steht,
mit Aminen der Formel III

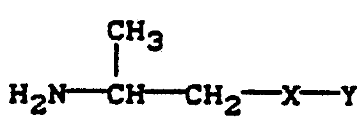 (III)

in welcher
X und Y die oben angegebene Bedeutung haben,
umsetzt und anschließend die Carbonylgruppe reduziert, oder

b) indem man Epoxide der Formel IV

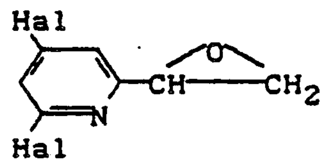

(IV)

in welcher
Hal für Halogen steht,
$R^1$, $R^2$, $R^3$ die oben angegebene Bedeutung haben,
mit Aminen der Formel III

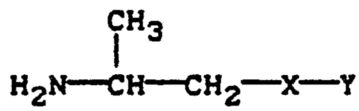

(III)

in welcher
X und Y die oben angegebene Bedeutung haben,
umsetzt, oder

c) indem man β-Halogenethylverbindungen der Formel V

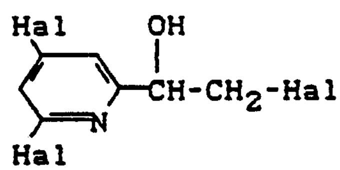

(V)

in welcher
Hal für Halogen steht,
mit Aminen der Formel III

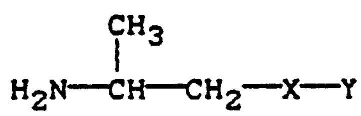

(III)

in welcher
X und Y die oben angegebene Bedeutung haben,
umsetzt, oder

d) Verbindungen der Formel VI

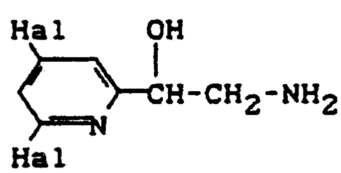

(VI)

in welcher
Hal für Halogen steht,
mit Ketonen der Formel VII

$CH_3-\overset{\overset{O}{||}}{C}-CH_2-X-Y$ (VII)

in welcher
X und Y die oben angegebene Bedeutung haben,

unter reduzierenden Bedingungen umsetzt, oder

e) indem man Verbindungen der Formel VIII

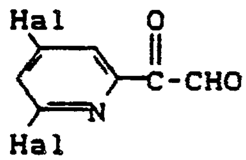

(VIII)

in welcher
Hal für Halogen steht,
mit Aminen der Formel III

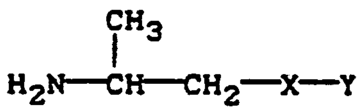

(III)

in welcher
X und Y die oben angegebene Bedeutung haben,
unter reduzierenden Bedingungen umsetzt, oder

f) indem man Verbindungen der Formel IX

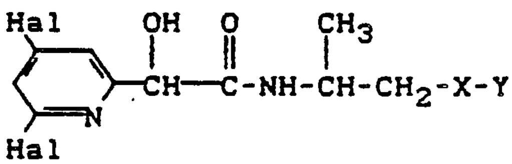

(IX)

in welcher
Hal, X und Y die oben angegebene Bedeutung haben,
reduziert.

3. Neue Verbindungen der Formel IX

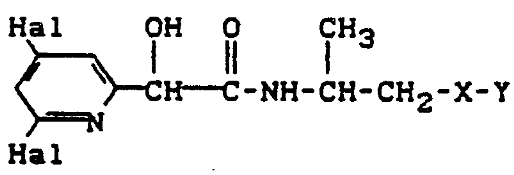

(IX)

in welcher
Hal, X und Y die bei den Verbindungen der Formel I angegebenen Bedeutungen haben.

4. Neue Verbindungen der Formel X

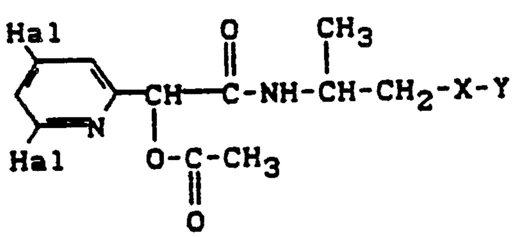

(X)

in welcher

Hal, X und Y die oben angegebene Bedeutung haben.

5. Neue Verbindungen der Formel II

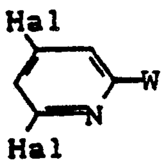

(II)

in welcher
Hal für Halogen steht und

W für 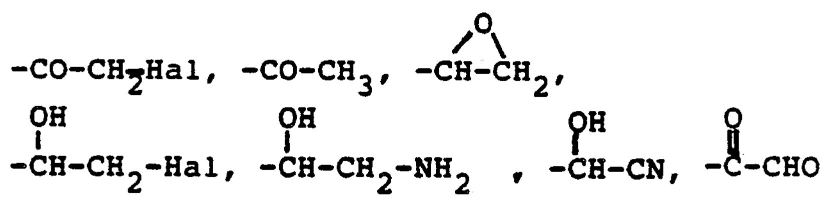

6. Mittel zur Verschiebung des Protein-Fett-Verhältnisses zugunsten von Protein sowie zur Behandlung der Adipositas bei Mensch und Tier, gekennzeichnet durch einen Gehalt an 2,4-Dihalogen-6-pyridylethanolphenylisopropylamine der Formel I gemäß Anspruch 1.

7. Tierfutter, Trinkwasser für Tiere, Zusätze für Tierfutter und Trinkwasser für Tiere, gekennzeichnet durch einen Gehalt an 2,4-Dihalogen-6-pyridylethanolphenylisopropylamine der Formel I gemäß Anspruch 1.

8. Verwendung von 2,4-Dihalogen-6-pyridylethanolphenylisopropylamine der Formel I gemäß Anspruch 1 zur Verschiebung des Protein-Fett-Verhältnisses zugunsten von Protein.

9. Verfahren zur Herstellung von Mitteln zur Verschiebung des Protein-Fett-Verhältnisses zugunsten von Protein sowie zur Behandlung der Adipositas bei Mensch und Tier, dadurch gekennzeichnet, daß man 2,4-Dihalogen-6-pyridylethanolphenylisopropylamine der Formel I gemäß Anspruch 1 mit Streck- und/oder Verdünnungsmitteln versetzt.

10. Verfahren zur Herstellung von Tierfutter, Trinkwasser für Tiere oder Zusätze für Tierfutter und Trinkwasser für Tiere, dadurch gekennzeichnet, daß man 2,4-Dihalogen-6-pyridylethanolphenylisopropylamine der Formel I gemäß Anspruch 1 mit Futtermitteln oder Trinkwasser und gegebenenfalls weiteren Hilfsstoffen vermischt.